# FASCICULE DE BREVET EUROPEEN

(11) **EP 3 526 553 B1**
(45) Date de publication et mention de la délivrance du brevet: **30.03.2022**
(21) Numéro de dépôt: 17794007.9
(22) Date de dépôt: 12.10.2017
(51) Int. Cl.: G01D 21/00

(54) **DISPOSITIF AUTONOME DE MESURE DES CARACTÉRISTIQUES D'UN FLUIDE CIRCULANT DANS UN CONDUIT ET SYSTÈME DE COMMANDE DE LA VENTILATION, DE LA CLIMATISATION ET/OU DU CHAUFFAGE UTILISANT UN TEL DISPOSITIF**
AUTONOME VORRICHTUNG ZUR MESSUNG DER EIGENSCHAFTEN EINES FLÜSSIGKEITSKREISLAUFS IN EINEM KANAL UND BELÜFTUNGS-, KLIMA- UND / ODER HEIZKONTROLLSYSTEM MIT EINER SOLCHEN ANLAGE
AUTONOMOUS DEVICE FOR MEASURING THE CHARACTERISTICS OF A FLUID CIRCULATING IN A DUCT AND VENTILATION, AIR CONDITIONING AND / OR HEATING CONTROL SYSTEM USING SUCH A DEVICE

(30) Priorité: 12.10.2016 FR 1659863
(43) Date de publication de la demande: 21.08.2019
(73) Titulaire: Enerbee, 38040 Grenoble (FR)
(72) Inventeur: DELAMARE, Jérôme, 38950 Quaix en Chartreuse (FR); VINCENT, Maxime, 38000 Grenoble (FR); LAVILLE, Jérôme, 38000 Grenoble (FR); RICART, Thibault, 38170 Seyssinet Pariset (FR); POUYADOU, Luc, 38190 Brignoud (FR); MAJ, Guillaume, 38410 Saint-Martin-D'Uriage (FR); AUMELAS, Vivien, 38130 Echirolles (FR); GUILAUMÉ, Samuel, 38040 Grenoble (FR)
(74) Mandataire: IP Trust
(86) Numéro de dépôt international: PCT/FR2017/052814
(87) Numéro de publication internationale: WO 2018/069656

(56) Documents cités:
- DE-A1-102007 012 946
- DE-A1-102010 028 793
- FR-A1- 2 824 907
- US-A1- 2009 134 631
- THOMAS LAFONT ET AL: "Paper;Magnetostrictive piezoelectric composite structures for energy harvesting;Magnetostrictive--piezoelectric composite structures for energy harvesting", JOURNAL OF MICROMECHANICS & MICROENGINEERING, INSTITUTE OF PHYSICS PUBLISHING, BRISTOL, GB, vol. 22, no. 9, 24 août 2012 (2012-08-24), pages 1-6, XP020228735, ISSN: 0960-1317, DOI: 10.1088/0960-1317/22/9/094009
- FLAMMINI A ET AL: "An autonomous sensor with energy harvesting capability for airflow speed measurements", INSTRUMENTATION AND MEASUREMENT TECHNOLOGY CONFERENCE (I2MTC), 2010 IEEE, IEEE, PISCATAWAY, NJ, USA, 3 mai 2010 (2010-05-03), pages 892-897, XP031691786, ISBN: 978-1-4244-2832-8
- XIANZHI DAI ET AL: "Energy harvesting from mechanical vibrations using multiple magnetostrictive/piezoelectric composite transducers", SENSORS AND ACTUATORS A: PHYSICAL, ELSEVIER BV, NL, vol. 166, no. 1, 31 décembre 2010 (2010-12-31), pages 94-101, XP028142137, ISSN: 0924-4247, DOI: 10.1016/J.SNA.2010.12.025 [extrait le 2011-01-04]

## Description

### DOMAINE DE L'INVENTION

La présente invention concerne un dispositif autonome de mesure des caractéristiques d'un fluide circulant dans un conduit, et notamment des caractéristiques du flux d'air circulant dans une gaine de ventilation, de climatisation ou de chauffage d'un bâtiment. L'invention concerne également un système de commande de la ventilation, de la climatisation et/ou du chauffage d'un bâtiment qui utilise un tel dispositif.

### ARRIERE PLAN TECHNOLOGIQUE DE L'INVENTION

Les bâtiments modernes sont tous équipés d'un système de ventilation, de climatisation et/ou de chauffage qui permet notamment de renouveler l'air intérieur et d'évacuer l'air vicié. On évite ainsi une accumulation d'une quantité excessive de composés ou de particules nocives : CO2, composés organiques volatils, etc. On évite également une humidité excessive qui peut conduire à la dégradation du bâtiment, et on confère un degré de confort satisfaisant pour les résidents.

Pour ce qui concerne la ventilation, et d'une manière générale, les solutions connues consistent à aspirer à travers des conduits ou des gaines l'air intérieur à partir des pièces susceptibles d'être les plus humides (salle d'eau, cuisine). De l'air frais provenant de l'environnement extérieur est introduit dans le bâtiment, soit naturellement à travers des ouvertures aménagées (généralement dans les pièces les plus sèches), soit par aspiration à travers des conduits permettant de prélever à l'extérieur une quantité d'air frais.

Quelle que soit la solution choisie, le renouvellement de l'air intérieur d'un bâtiment est une source de perte d'énergie. L'air extérieur qui vient renouveler l'air intérieur présente généralement des caractéristiques, notamment en température, bien différentes de l'air intérieur. Cela conduit, selon le cas, à un refroidissement ou un réchauffement du bâtiment qu'il faut compenser en utilisant des moyens complémentaires de chauffage ou de climatisation pour maintenir la qualité de l'air et le confort intérieur.

Dans ce contexte, des solutions intégrées sont envisagées et visent à traiter dans leur globalité les questions liées au chauffage, à la ventilation et la climatisation d'un bâtiment dans le but de fournir une qualité d'air intérieur et un confort attendu tout en maintenant la dépense énergétique la plus faible.

Ces solutions intégrées nécessitent la mise en place de dispositifs de mesure (capteurs de température, d'humidité, de qualité de l'air, etc.) afin de collecter de données caractérisant l'état interne du bâtiment. Les dispositifs de mesure sont reliés à un dispositif de commande, par exemple un calculateur, de sorte à pouvoir commander les moyens de chauffage, de ventilation et de climatisation. Le dispositif de commande a également pour fonction d'élaborer les lois de commandes de ces différents moyens permettant d'atteindre, avec la dépense énergétique la plus faible, les consignes de confort fournies par un résident ou un gérant du bâtiment et permettant de respecter les contraintes de qualité d'air.

Une collecte de données fines et représentatives de l'état du bâtiment nécessite la mise en place d'un nombre suffisant de dispositifs de mesure, et une répartition homogène de ces dispositifs dans le bâtiment. Cette mise en place, l'alimentation en énergie des dispositifs, les liaisons nécessaires avec le dispositif de commande pour pouvoir communiquer les données mesurées nécessitent des travaux couteux d'aménagement du bâtiment, notamment lorsqu'il s'agit d'équiper un bâtiment déjà existant. Cela représente un frein important au déploiement de telles solutions intégrées.

Pour remédier à ce problème, il serait bénéfique de disposer de dispositifs de mesure autonome en énergie et en communication. Ainsi, le document « An autonomous sensor with energy harvesting capability for airflow speed measurements », Instrumentation and Measurement Technology, Conference (I2MTC), 2010 IEEE propose un dispositif de mesure recherchant une telle autonomie. Plus précisément, ce document divulgue un dispositif de mesure de la vitesse de l'air circulant dans un conduit de ventilation. Ce dispositif comprend un ventilateur mis en mouvement par le flux d'air du conduit, et relié à une dynamo transformant le mouvement de rotation du ventilateur en une charge électrique. Cette charge est utilisée pour fournir une mesure du flux de l'air circulant dans le conduit. Ce document précise également que l'efficacité d'une éolienne augmente avec son paramètre de rapidité (également désigné par paramètre d'avance ou encore vitesse spécifique, « Tip Speed Ratio » selon l'expression anglo-saxonne) jusqu'à une valeur limite théorique de 59%, et incite donc à mettre en rotation rapide l'éolienne.

La solution de laboratoire exposée dans ce document ne convient toutefois pas pour un bâtiment conventionnel. Elle nécessite en effet une vitesse de flux d'air importante, supérieur à 3 m/s, pour fournir l'énergie suffisante à son fonctionnement. Or, conventionnellement, le flux d'air de ventilation peut présenter une vitesse aussi faible que 0,1 m/s. Le dispositif présenté dans le document précité ne peut fonctionner dans ces conditions.

On notera également que pour des raisons de confort, on favorise l'installation de conduits de ventilation, de chauffage, ou de climatisation présentant une section importante de sorte faire circuler, pour un débit donné, un flux d'air à plus faible vitesse. Ce flux peut ainsi atteindre une fraction de m/s. On limite de la sorte les émissions sonores liées à la circulation à grande vitesse du fluide dans le conduit. Et on peut choisir des dispositifs de mise en circulation du fluide dans les conduits (pompe ou moteur d'extraction ou de propulsion) moins puissants et donc moins consommateurs en énergie. Le dispositif présenté dans le document précédent n'est donc aucunement adapté aux installations de chauffage, de ventilation et de climatisation d'un bâtiment moderne.

De plus, le dispositif de mesure proposé par ce document prévoit de transmettre les mesures de vitesse collectées à un récepteur. Pour compenser la faible quantité d'énergie collectée, c'est le récepteur lui-même qui fournit l'énergie nécessaire à cette transmission, par rayonnement électromagnétique. Le récepteur doit donc être positionné suffisamment proche du dispositif de mesure pour permettre la fourniture efficace de l'énergie, ce qui représente une contrainte importante.

On connaît également du document DE102010028793 un dispositif autonome en énergie permettant la mesure des caractéristiques d'un fluide circulant dans un conduit. Ce document reconnaît la difficulté de mettre en mouvement une turbine d'un dispositif de récupération d'énergie lorsque la vitesse du fluide dans le conduit et faible. Il préconise d'augmenter la vitesse du fluide, par exemple en restreignant localement le diamètre du conduit, et permettre ainsi le fonctionnement du dispositif de récupération d'énergie. Toutefois, dans un tel mode de fonctionnement « rapide » de la turbine, l'usure et l'émission sonore du dispositif sont exacerbées.

On connaît enfin du document « Magnetostrictive piezoelectric composite structures for energy harvesting » de T. Lafont et Al, parrus dans Journal of Micromechanics & Microengineering, Institute of Physics publishing, Vol 22, n°9, 24-08-2012 une technologie de récupération d'énergie couplant des matériaux magnétostrictifs et piézoélectriques.

### OBJET DE L'INVENTION

La présente invention vise à pallier au moins en partie les problèmes exposés ci-dessus. Elle vise notamment à fournir un dispositif de mesure d'une caractéristique d'un fluide circulant dans un conduit, autonome en énergie. Elle vise également à fournir un système de commande de la ventilation, de la climatisation et/ou chauffage d'un bâtiment qui soit d'installation très simple et très peu couteuse. Plus particulièrement encore, la présente invention vise un dispositif de mesure d'une caractéristique d'un fluide circulant dans un conduit qui présente une durée de fonctionnement étendue et qui présente en fonctionnement une émission sonore faible.

### BREVE DESCRIPTION DE L'INVENTION

En vue de la réalisation de l'un de ces buts, l'objet de l'invention propose un dispositif autonome de mesure d'au moins une caractéristique d'un fluide circulant dans un conduit selon une direction d'écoulement, le dispositif étant défini par la revendication 1.

Selon l'invention, l'un du convertisseur et de la source sont compris dans le stator, et l'autre est compris dans le rotor. Le stator est assemblé au rotor de sorte que le plan de référence du convertisseur réside dans le plan magnétique généré par la source.

Selon d'autres caractéristiques avantageuses et non limitatives de l'invention, prises seules ou selon toute combinaison techniquement réalisable :
- la source est comprise dans le rotor, solidaire de la turbine ; et le convertisseur est compris dans le stator ;
- le convertisseur et le circuit de traitement sont compris dans le rotor, solidaire de la turbine ; et la source est comprise dans le stator ;
- le convertisseur et le circuit de traitement sont disposés dans un boîtier étanche ;
- les pales sont orientables ou constituées d'un matériau flexible ;
- le stator est au moins partiellement logé dans le moyeu de la turbine.
- le moyeu présente une forme de dôme creux et de profil aérodynamique, afin d'accélérer l'écoulement du fluide vers les pales ;
- le moyeu comprend un rideau susceptible de dégager un passage de circulation du fluide ;
- la turbine est conçue pour présenter un paramètre de rapidité inférieure à trois ;
- la turbine présente une solidité comprise entre 0,7 et 1, et préférentiellement proche de 1.
- l'angle de calage des pales est compris entre 15° et 45° ;
- l'angle de calage des pales est variable ;
- le convertisseur comprend dans son plan de référence une couche de matériaux magnétisable, et magnétiquement retenue dans le plan magnétique généré par la source de sorte à former un assemblage démontable entre le rotor et le stator ;
- un arbre de rotation aligné avec l'axe de rotation de la turbine est disposé entre un centreur placé sur le rotor et un centreur placé sur le stator pour guider la rotation du rotor ;
- la source est formée par une pluralité d'aimants configurés en cylindre de Halbach; l'axe du cylindre étant aligné avec l'axe de rotation de la turbine ;
- les moyens de fixation comprennent une bouche de conduit, le rotor et le stator étant placés à l'intérieur de la bouche de conduit ;
- la turbine est munie d'un identifiant de turbine ;
- la mesure d'une caractéristique d'environnement fournie par le circuit de traitement comprend l'un au moins de la vitesse du fluide, le débit du fluide, la température du fluide, la concentration en CO2, la concentration en CO, la concentration en composés organiques volatiles, le degré d'humidité du fluide ;
- le dispositif comprend au moins un capteur choisi parmi un capteur de température, un capteur de concentration en CO2, un capteur de concentration en CO ou en particules organiques volatiles, un capteur d'humidité, un détecteur d'identifiant de turbine ;
- le circuit de traitement comprend un circuit de prélèvement des charges ;
- le circuit de traitement comprend un circuit de transmission de la mesure ;
- le circuit de traitement comprend un dispositif de stockage des charges électriques ;

Selon un autre aspect, l'invention porte sur un système de commande de la ventilation, de la climatisation et/ou du chauffage d'un bâtiment, le bâtiment comportant un réseau de conduits, le système comportant :
- au moins un dispositif autonome de mesure ;
- un dispositif de commande configuré pour traiter la mesure fournie par le dispositif autonome et ajuster l'état de ventilation, de climatisation et/ou de chauffage du bâtiment.

Selon d'autres caractéristiques avantageuses et non limitatives de l'invention, prises seules ou selon toute combinaison techniquement réalisable :
- le dispositif de commande est également configuré pour fournir des informations de maintenance ou de diagnostic du réseau de conduits ;
- le dispositif de commande est également configuré pour configurer les paramètres de fonctionnement du dispositif autonome ;
- le système de commande comprend également des capteurs de bâtiment additionnels, tels que des capteurs de température d'une pièce ou de présence dans une pièce ;
- le dispositif autonome comprend des moyens pour modifier le débit de fluide circulant dans le conduit dans lequel il réside ;
- le dispositif de commande est au moins en partie intégré au dispositif autonome pour former un dispositif auto régulé ;
- le système de commande comprend une pluralité de dispositifs autonomes auto régulés configurés pour communiquer entre eux ;
- le dispositif de commande est un calculateur, tel qu'un téléphone intelligent, une tablette ou un ordinateur ;
- le dispositif de commande est distant du dispositif autonome, et la mesure fournie par le dispositif autonome est communiquée au dispositif de commande par un réseau longue distance, tel que le réseau SigFox ou Internet.

### BREVE DESCRIPTION DES DESSINS

D'autres caractéristiques et avantages de l'invention ressortiront de la description détaillée de l'invention qui va suivre en référence aux figures annexées sur lesquels :
- les figures la à 1c représentent un générateur selon différents modes de mise en œuvre de l'invention ;
- les figures 2a et 2b représentent une coupe schématique et une vue de dessus d'un convertisseur ;
- la figure 2c est une représentation graphique de la quantité de charges générées par le convertisseur en fonction de l'angle θ existant entre la direction du champ magnétique et la direction de polarisation des couches piézoélectriques ;
- les figures 3a et 3b sont respectivement une vue éclatée et une coupe d'un dispositif autonome conforme à l'invention ;
- la figure 4 est un graphe reliant la vitesse d'écoulement de l'air dans un conduit de ventilation à la vitesse de rotation du rotor et à la puissance générée par le générateur ;
- les figures 5a à 5e représentent des turbines pouvant être mise en œuvre dans un dispositif conforme à l'invention ;
- la figure 6 est un graphe donnant la vitesse rotation d'une turbine d'un dispositif conforme à l'invention suivant la vitesse du fluide circulant dans un conduit.

### DESCRIPTION DETAILLEE DE L'INVENTION

Par souci de simplification de la description à venir, les mêmes références sont utilisées pour des éléments identiques ou assurant la même fonction dans les différents modes de mise en œuvre de l'invention.

La présente invention porte sur un dispositif autonome pour mesurer au moins une caractéristique d'un fluide circulant dans un conduit et éventuellement en modifier une ou plusieurs caractéristiques. Le dispositif est autonome en énergie, et à ce titre, il comprend un générateur qui transforme l'énergie cinétique du fluide en des charges électriques. Les principes de fonctionnement du générateur sont exposés ci-dessous.

### Générateur

Comme cela est représenté à titre d'exemple sur les figures la à 1c, le générateur 1 exploité par l'invention comprend un convertisseur magnéto-électrique 2 et une source de champ magnétique 3, tel qu'un aimant permanent. Le convertisseur 2 et la source 3 peuvent se déplacer l'un par rapport à l'autre. Comme cela sera exposé dans le détail dans la suite de cet exposé, le générateur peut être maintenu fixe, et la source 3 peut être mise en rotation par une turbine entrainée par la circulation du fluide. Avantageusement, la source 3 définit un logement 4 dans lequel le convertisseur 2 peut être placé et former un ensemble particulièrement compact. Les charges fournies par le générateur sont collectées, et éventuellement stockées à l'aide d'un circuit de prélèvement associé au générateur 1. Un tel circuit est connu par exemple de WO2014063958, WO2014063952, WO2014063951, WO2016146929 ou WO2007063194 et ne sera pas décrit ici plus en détail.

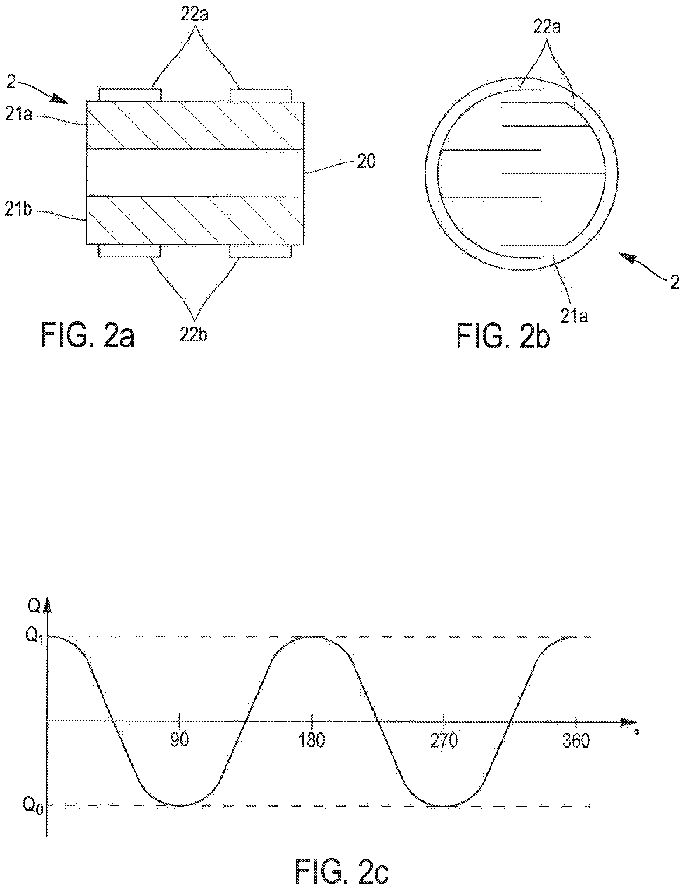

Les figures 2a et 2b représentent un exemple particulier d'un convertisseur magnéto-électrique 2. Le convertisseur 2 est apte à transformer la variation d'un champ magnétique dans un plan de référence en une déformation mécanique susceptible de générer une accumulation de charges.

Le convertisseur 2 comprend une couche 20 de matériau magnétostrictif présentant préférentiellement un coefficient de magnétostriction, en valeur absolue et à saturation, supérieur à 10 ppm, ou supérieur à 30 ppm voire même à 100 ou à 1000 ppm. On rappelle que ce coefficient est défini par le quotient ΔL/L où ΔL est l'élongation du matériau en présence d'un champ magnétique saturant le matériau, et L la longueur de ce matériau en l'absence de champ magnétique.

De préférence, le matériau de la couche 20 est choisi pour être intrinsèquement isotrope ou présenter un comportement isotrope dans le générateur 1. Il peut être par exemple formé d'un bloc de Terfenol-D cristallin ou fritté, de Galfenol, de Terbium Fer, de Fer-Cobalt, de Fer-Nickel ou encore de FeSiB amorphe.

Comme cela est visible en figure 2b, sur la vue de dessus du convertisseur 2, la couche 20 peut avoir une forme de disque. La couche 20 définit un plan de référence pour le convertisseur 2 et le générateur 1 dans lequel il est placé.

Comme cela est bien connu en soi, l'application d'un champ magnétique à la couche 20 selon une direction déterminée dans le plan de référence entraîne la déformation de la couche selon cette direction déterminée (une élongation lorsque le coefficient de magnétostriction de la couche 20 est supérieur à 0) .

Le convertisseur 2 magnéto-électrique comporte également, assemblé solidairement à la couche 20, au moins une couche piézo-électrique 21a, présentant des électrodes 22a. Sur l'exemple représenté sur la figure 2a, deux couches piézoélectriques 21a, 21b sont assemblées sur l'une et l'autre des faces de la couche 20. Chacune de ces couches piézo-électriques 21a, 21b disposent d'électrodes 22a, 22b au moins sur l'une de leurs faces, par exemple sur leur face libre. Comme cela est visible sur la figure 2b pour ce qui concerne l'électrode 22a, les électrodes 22a, 22b peuvent être interdigitées afin de collecter efficacement les charges de chacune des couches 21a, 21b.

La (ou les) couche piézoélectrique 21a, 21b est préférentiellement préparée pour fonctionner en mode d₃₃, qui est avantageusement plus sensible que le mode d₃₁. Cela contribue à améliorer les performances du convertisseur. Comme les couches piézoélectriques 21a, 21b sont solidairement assemblées à la couche magnétostrictive 20, la déformation de cette couche 20 dans le plan de référence entraîne également la déformation des couches piézo-électriques 21a, 21b dans un plan parallèle à ce plan de référence.

Les couches piézoélectriques 21a, 21b sont préférentiellement polarisées selon une direction de polarisation contenue dans le plan qu'elles définissent. Lorsque plusieurs couches piézo-électriques 21a, 21b sont présentes, elles sont avantageusement disposées sur la couche magnétostrictive 20 pour que leurs axes de polarisation soient disposés parallèles l'un à l'autre. On considèrera que c'est bien le cas dans la description venir.

La déformation des couches piézoélectriques (21a), (21b) selon leurs directions de polarisation entraîne une création de charges électriques dans ces couches et leur accumulation sur les électrodes (22a), (22b). Une telle déformation est obtenue lorsque la couche magnétostrictive (20) est soumise à un champ magnétique dont l'orientation présente une composante parallèle à la direction de polarisation des couches (21a), (21b).

La figure 2c est une représentation graphique de la quantité de charges générées sur les électrodes 22a, 22b en fonction de l'angle θ existant entre la direction d'un champ magnétique uniforme se développant dans la couche 20 magnétostrictive et la direction de polarisation des couches 21a, 21b. On observe que, en l'absence de leurs collectes, les charges accumulées oscillent entre une valeur maximale Q1 et une valeur minimale Q0. La valeur maximale est atteinte lorsque l'angle θ est égal à 0° et 180°, c'est-à-dire lorsque les directions du champ magnétique et de l'axe de polarisation sont parallèles. La valeur minimale Q0 est atteinte lorsque l'angle θ égal 90° et 270°, c'est-à-dire lorsque la direction du champ magnétique et de l'axe de polarisation sont perpendiculaires. Entre deux extrêmes consécutifs, il y a donc création de charges (positives ou négatives) dans les couches piézoélectriques 21a, 21b.

Avantageusement, lorsque le convertisseur 2 est soumis à un champ magnétique tournant, le circuit de prélèvement est configuré pour collecter les charges créées à chaque quart de tour, pour des angles θ de 0°,90°, 180° et 270°.

On pourra, selon le besoin, omettre au moins une de ces étapes de collecte de quart de tour, ou décaler angulairement ces étapes de collecte, par exemple pour réduire le couple de freinage induit, notamment lorsque suffisamment d'énergie est disponible ou bien lorsque le couple d'entrainement est faible, tel que dans les bas débits d'air tel que cela sera détaillé ultérieurement.

On forme de la sorte un convertisseur 2 magnéto-électrique apte à transformer les variations, dans le plan de référence définie par la couche 20 magnétostrictive, d'un champ magnétique en une déformation mécanique susceptible de générer une accumulation de charges au niveau d'au moins une électrode 22a, 22b d'au moins une couche piézo-électrique 21a, 21b. On pourra connecter l'électrode 22a, 22b à une borne à laquelle on pourra connecter le circuit de prélèvement.

On notera que le générateur n'est nullement limité à un convertisseur 2 de la forme précise qui vient d'être décrite. Ainsi un convertisseur 2 comprenant une unique couche piézoélectrique 21a ou comprenant une pluralité de couches magnétostrictives est tout à fait compatible avec l'invention. De même, les électrodes 22a, 22b peuvent prendre d'autres formes ou être déployées différemment de ce qui a été décrit dans les paragraphes précédents.

Revenant à la description des figures la à 1c, le générateur 1 comprend également une source de champ magnétique 3. La source de champ magnétique 3 peut définir un logement 4 dans lequel règne un champ magnétique. Sur les figures la à 1c ce champ est représenté par les lignes de champ en traits pointillés. Pour limiter l'encombrement du générateur 1 (par exemple de l'ordre du centimètre cube, ce qui est parfaitement compatible avec les applications visées par la présente demande), on cherche à réduire les dimensions de la source de champ magnétique 3. L'intensité du champ peut alors être choisie de l'ordre de 0,3 Tesla, ou compris entre 0,03 et 0,6 Tesla.

Préférentiellement, le logement 4 et la source 3 sont configurés pour que le convertisseur 2 puisse être placé dans le logement de manière à disposer une partie au moins du champ dans son plan de référence. La source 3 et le convertisseur 2 sont libres de se déplacer l'un par rapport à l'autre, afin de pouvoir créer dans le logement 4 un champ tournant vis-à-vis du convertisseur.

Préférentiellement, le champ régnant dans le logement 4 est uniforme, c'est-à-dire qu'il présente une direction et/ou une intensité relativement constante au moins dans une partie centrale du logement et préférentiellement en tout point du logement. Il est ainsi aisé de placer le convertisseur dans le logement 4 sans avoir le besoin de le positionner avec précision en un lieu particulier.

Il existe de multiples manières de réaliser la source de champ 3.

Selon une première approche, la source 3 est formée d'un assemblage plan d'aimants permanents orientés les uns par rapport aux autres de manière à confiner un champ magnétique d'un côté de ce plan. Cet assemblage est bien connu sous le nom de « réseau d'Halbach ».

En plaçant en vis-à-vis deux de ces assemblages, les champs se faisant face, on définit le logement 4 par l'espace séparant ces deux plans. Cette configuration est représentée en figure la. On notera toutefois qu'il n'est pas nécessaire de disposer de deux assemblages plans, et qu'un unique assemblage est suffisant pour générer un champ magnétique utile.

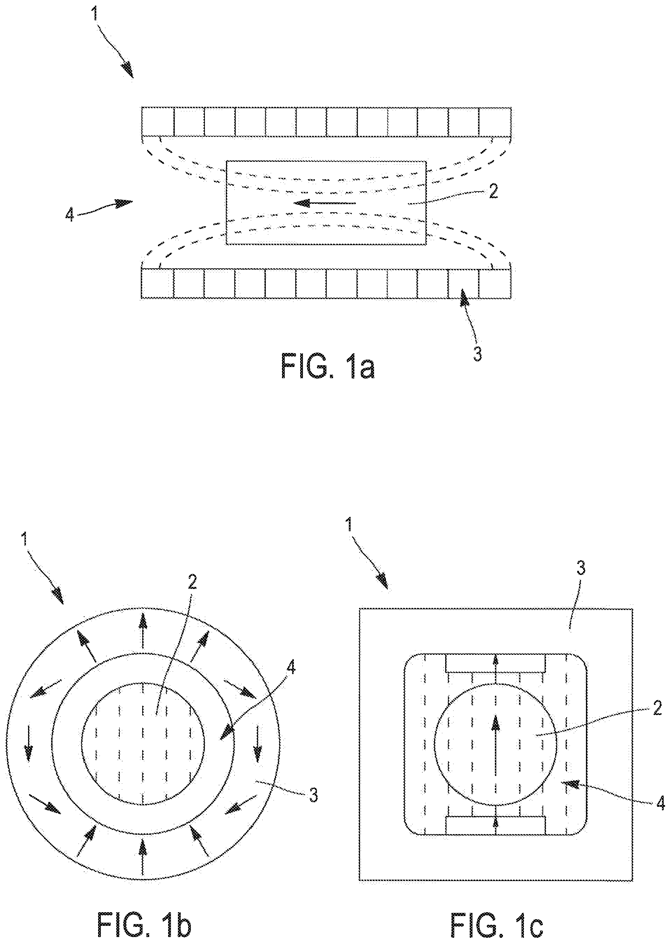

Selon une seconde approche, une pluralité d'aimants permanents sont disposés les uns par rapport aux autres le long d'un contour fermé pour définir le logement 4 et créer un champ à l'intérieur de celui-ci. À titre d'exemple, il peut s'agir d'une configuration en cylindre de Halbach, représentée schématiquement sur la figure 1b.

À titre d'exemple complémentaire, il peut s'agir d'une structure fermée en fer doux, définissant le logement, deux aimants permanents de moment magnétique identique sont placés en vis-à-vis dans le logement comme cela est représenté sur la figure 1c.

Quelle que soit la configuration choisie de la source 3, le convertisseur 2 est placé vis à vis de la source pour qu'une partie au moins du champ soit disposée dans le plan de référence.

La présente invention exploite les principes technologiques qui viennent d'être présentés pour proposer un dispositif autonome de mesure d'une caractéristique d'un fluide circulant dans un conduit.

### Utilisation du générateur pour la récupération de l'énergie d'un fluide circulant dans un conduit de ventilation.

Dans certains exemples particuliers qui vont être exposés, le générateur est utilisé pour récupérer l'énergie d'un fluide circulant dans un conduit de ventilation d'un bâtiment. Ce fluide est donc de l'air. On notera toutefois que l'invention n'est certainement pas limitée à ce type de conduit et de fluide.

L'air peut être mis en circulation dans le conduit par aspiration ou par propulsion. On utilisera l'expression « direction d'écoulement » dans la suite de l'exposé pour désigner de manière générale la direction de l'écoulement du fluide dans la section de conduit dans lequel le dispositif est placé.

Dans l'exemple particulier des conduits de ventilation d'un bâtiment, ceux-ci peuvent se présenter sous la forme d'une gaine flexible de section circulaire et de diamètre normalisé de 50mm, 90mm, 125mm ou 300mm. Mais un dispositif conforme à l'invention n'est pas lié à des dimensions ou à une forme particulière du conduit.

Le débit de ventilation usuel d'un bâtiment ou d'un logement résidentiel peut être compris entre 50 m³ par heure et 200 m³ par heure lorsqu'il n'est pas volontairement contrôlé à une valeur inférieure. Des normes peuvent ainsi imposer un débit particulier selon la nature des pièces, par exemple un débit de 50m³ par heure pour la ventilation d'une cuisine ou de 15m³ par heure pour la ventilation de toilettes. La vitesse de l'air qui s'écoule dans les conduits peut être comprise entre une fraction de mètre par seconde, comme de 0,05 m/s ou 0,1m/s ou encore 0,5m/s à plusieurs mètres par seconde comme 4m/s, 6m/s ou 10 m/s.

Un dispositif conforme à l'invention est opérationnel dans une large gamme de vitesse d'air, et idéalement sur toute la gamme comprise entre 0,05m/s à 10 m/s, comme par exemple dans la gamme comprise entre 0,lm/s à 10 m/s, ou entre 1 m/s et 10 m/s ou encore entre 0,1 m/s et 4 m/s.

Comme cela sera détaillé par la suite, un dispositif autonome selon l'invention extrait son énergie de fonctionnement de l'énergie d'écoulement du fluide dans le conduit, à l'aide d'une turbine. Par unité de volume, cette énergie correspond à la différence de pression **Δ**p engendrée côté fluide de part et d'autre de la turbine. De façon complémentaire, la puissance instantanée disponible côté fluide prend la forme Q * **Δ**p**,** où Q est le débit volumique du fluide égal au produit U * S, où U est la vitesse moyenne d'écoulement de l'air dans le conduit de section S. On voit que pour une section donnée, la puissance instantanée disponible est fortement dépendante de la vitesse du fluide. La faculté de fonctionner sur une large gamme de vitesse de fluide, et notamment pour les vitesses de fluide les plus basses est une caractéristique très avantageuse de l'invention.

### Dispositif autonome de mesure

D'une manière générale, un dispositif autonome de mesure conforme à l'invention combine des moyens permettant de récupérer l'énergie du fluide circulant dans le conduit et des moyens de traitement permettant de fournir une mesure d'au moins une caractéristique de ce fluide. L'énergie récupérée, à l'aide du générateur dont les principes de fonctionnement ont été présentés dans les paragraphes précédents, est suffisante pour alimenter en énergie toute la chaîne de traitement nécessaire à la fourniture de cette mesure. Les moyens sont suffisamment compacts pour être placés dans le conduit ou à l'une de ses extrémités.

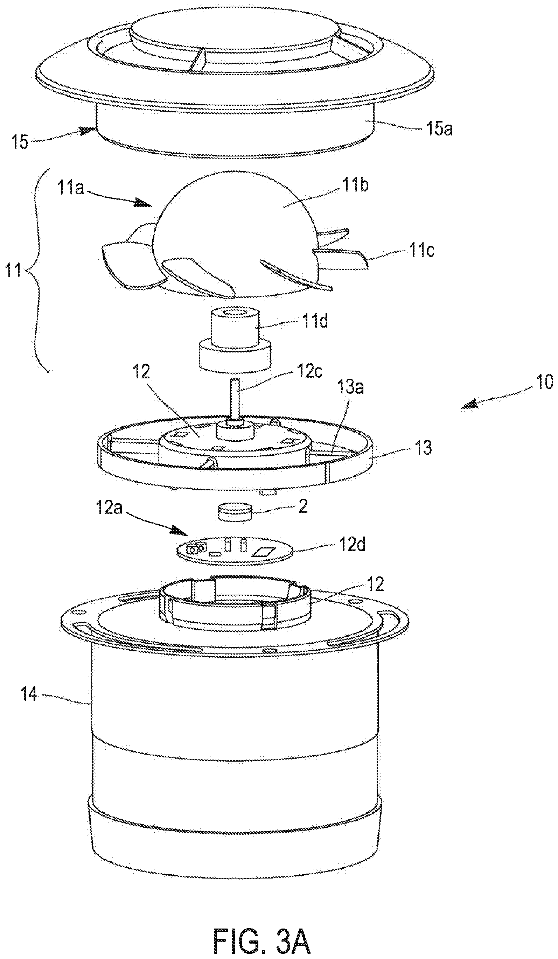

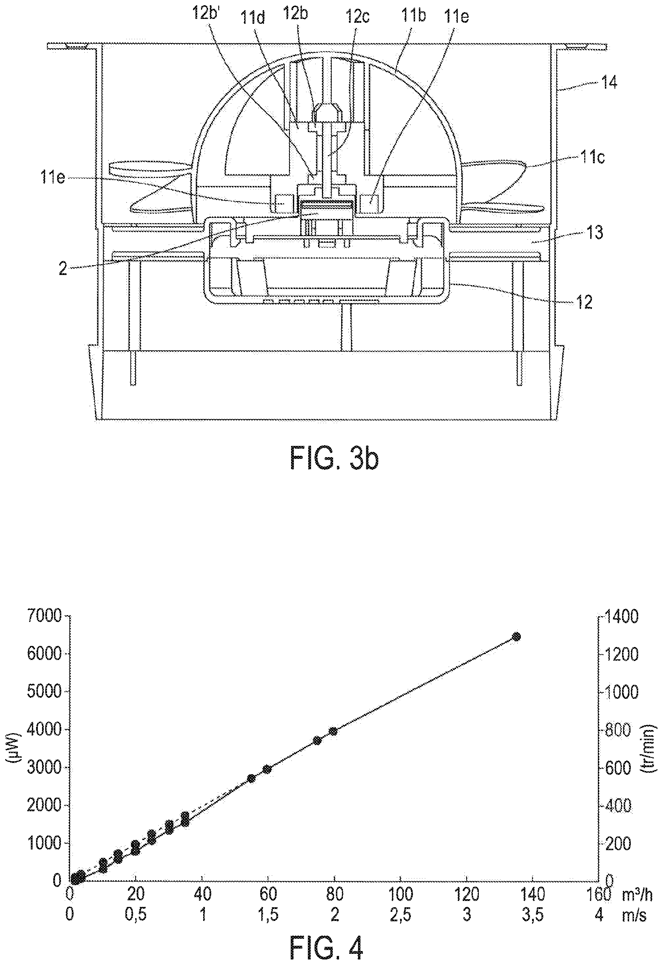

Ainsi, et comme cela est représenté en vue éclatée sur la figure 3a, et en coupe sur la figure 3b, un dispositif autonome 10 conforme à l'invention se présente sous la forme d'un assemblage comprenant un rotor 11, un stator 12, et de moyens de fixation 13 permettant de placer fixement le stator dans un conduit 14, ou à une extrémité de ce conduit, et d'orienter le dispositif dans la direction d'écoulement.

L'invention prévoit indifféremment de placer le convertisseur 2 dans le stator 12, et la source 3 dans le rotor ou de placer le convertisseur 2 dans le rotor et la source 3 dans le stator. En d'autres termes, l'un du convertisseur 2 et de la source 3 est compris dans le stator 12, et l'autre est compris dans le rotor 11. Par souci de simplification, on se contentera de fournir une description détaillée d'un seul de ces modes de réalisation.

### Le rotor

Le rotor 11 comprend une turbine 11a qui présente un axe de rotation. La turbine a pour fonction de mettre en mouvement de rotation le rotor 11. Lorsque le dispositif autonome est convenablement assemblé et maintenu dans le conduit 14 par les moyens de fixation, l'axe de rotation de la turbine est parallèle à la direction d'écoulement. La turbine 11a est dès lors susceptible d'être entraînée en rotation par le fluide circulant dans le conduit 14, et elle est susceptible de mettre en mouvement le rotor 11.

La turbine 11a est conçue pour générer un couple supérieur au couple résistif, statique et dynamique, qui s'oppose à son mouvement, même à très faible vitesse de fluide, par exemple inférieure à 1m/s. Ce couple résistif peut comprendre un couple de friction trouvant son origine dans les éventuels frottements du rotor 11 avec son environnement lorsqu'il est en mouvement. Le couple résistif peut également comprendre un couple de freinage résultant de la conversion d'énergie qui se produit dans le stator 12.

De manière avantageuse, la turbine 11a est ainsi conçue pour former une turbine lente, c'est-à-dire que son paramètre de rapidité w.r/v (où w est la vitesse de rotation angulaire de la turbine, r son rayon, et v la vitesse d'écoulement du fluide) est inférieur à 3. On dispose ainsi d'une turbine 11a qui favorise le développement d'un couple plutôt que l'obtention d'une vitesse de rotation importante, et dédiée à l'exploitation des très faibles débits.

D'une manière plus générale, les formes aérodynamiques de la turbine 11a sont conçues pour favoriser le couple aérodynamique à bas débit tout en limitant, de plus, la vitesse de rotation de la turbine lors de son fonctionnement.

Il est alors possible de mettre en mouvement la turbine 11 pour une vitesse de fluide réduite, comparée à la vitesse de fluide nécessaire pour entrainer une turbine présentant un paramètre de rapidité supérieure à 3. De plus, une vitesse de rotation relativement faible de la turbine 11a (et donc du rotor 11) permet de limiter l'émission sonore du dispositif autonome 10, ce qui est un paramètre important lorsque celui-ci doit être disposé dans un conduit de ventilation débouchant sur un bureau ou une chambre. Enfin, une vitesse de rotation faible limite également l'usure par frottement des pièces formant le dispositif. On notera que le choix d'une turbine adaptée aux faibles vitesses d'air, et présentant notamment un paramètre de rapidité inférieur à 3, est tout à fait à l'encontre de l'enseignement qui découle des documents cités de l'état de la technique qui recherchent tous, par différent moyens, à placer le générateur dans un mode de fonctionnement correspondant à une vitesse de rotation rapide du rotor, afin de rendre possible ou efficace la conversion énergétique en régime établi à l'aide des générateurs conventionnels décrits.

La vitesse de rotation de la turbine 11a et du rotor 11 sont bien entendues dépendantes de la vitesse de circulation du fluide. Pour une vitesse relativement basse, de l'ordre de 1 m/s ou moins pour de l'air circulant dans un conduit de ventilation, il est possible de mettre en rotation le rotor à une vitesse angulaire de l'ordre de 10 à 100 tr/min, ce qui est suffisant pour fournir l'énergie de fonctionnement dispositif. D'une manière générale, on cherche à configurer la turbine 11a du rotor 11 pour obtenir une vitesse de rotation comprise entre 60 tr/min à 1000tr/min, préférentiellement entre 400 tr/min et 800 tr/min ou 600tr/min, sur une plage étendue de vitesse d'air dans le conduit (par exemple entre 0,1 m/s et 4 m/s). Dans ces gammes de vitesse de rotation, l'énergie récupérée est suffisante pour rendre autonome le dispositif, et suffisamment faible pour limiter l'usure et l'émission sonore de ce dispositif. Outre la conception adaptée de la turbine, et comme cela sera expliqué plus en détail dans la suite de cet exposé, la recherche d'une telle vitesse de rotation, relativement faible, peut conduire à dériver une partie du débit d'air du conduit pour qu'il ne contribue pas à la mise en rotation de la turbine, afin de limiter sa vitesse pour les plus grandes vitesses d'air notamment. Enfin, et comme cela sera également relevé dans la suite de cet exposé, on note que les mécanismes mis en œuvre dans le générateur d'un dispositif conforme à l'invention est particulièrement efficace pour la récupération de l'énergie fourni par un mouvement lent et/ou de faible amplitude.

Comme cela est bien visible sur la figure 3a la turbine 11a présente une architecture très conventionnelle. Elle est formée d'un moyeu central 11b et d'une pluralité de pales 11c fixées sur ce moyeu 11b. Cette architecture conventionnelle dispose de propriétés très bien établies.

Avantageusement, le moyeu 11a présente une forme de dôme creux de profil aérodynamique. Le dôme présente un sommet et une base qui peut être circulaire. Le sommet du dôme est orienté dans le conduit 14 vers l'amont de l'écoulement, afin de tirer profit du profil aérodynamique. On tente ainsi de préserver un écoulement essentiellement laminaire du fluide dans le conduit 14, et on évite de la sorte les pertes excessives de charges dans ce conduit. On profite également du profil aérodynamique du moyeu 11b pour accélérer l'écoulement du fluide vers les pales. Le diamètre du moyeu peut être choisi pour être compris entre 30% et 90% du diamètre de la turbine 11a (c'est-à-dire de la distance séparant l'extrémité de deux pales 11c diamétralement opposées).

Avantageusement également, la turbine 11a présente une solidité comprise entre 0,7 et 1. On rappelle que la solidité d'une turbine est la proportion de la surface du disque balayé par la turbine qui est occupée par la surface des pales et du moyeu. Une solidité importante, proche de 1, favorise le développement d'un couple important, et constitue un choix de conception privilégié. On peut néanmoins choisir une solidité moindre, par exemple proche de 0,7, pour que la présence du dispositif autonome 10 dans le conduit 14 n'affecte pas trop l'écoulement du fluide, et ne conduise à des pertes excessives de charge dans le conduit 14.

De manière préférée, les pales 11c sont fixées sur le moyeu 11b pour présenter un angle de calage compris entre 15° et 45°. On contribue de la sorte à former une éolienne lente. D'une manière très générale en effet, plus l'angle de calage est important plus la vitesse de rotation est faible. Par souci de clarté, on rappelle que l'angle de calage se définit comme l'angle que forme une pale avec le plan engendré par la rotation des pales. Lorsque la pale présente un profil d'aile, cet angle de calage est celui que forme la corde de ce profil avec le plan engendré par la rotation des pales.

Selon une variante très avantageuse, on peut prévoir de rendre cet angle de calage variable et, éventuellement, contrôlable, par exemple par l'intermédiaire des moyens de traitement qui seront détaillés dans la suite de cet exposé.

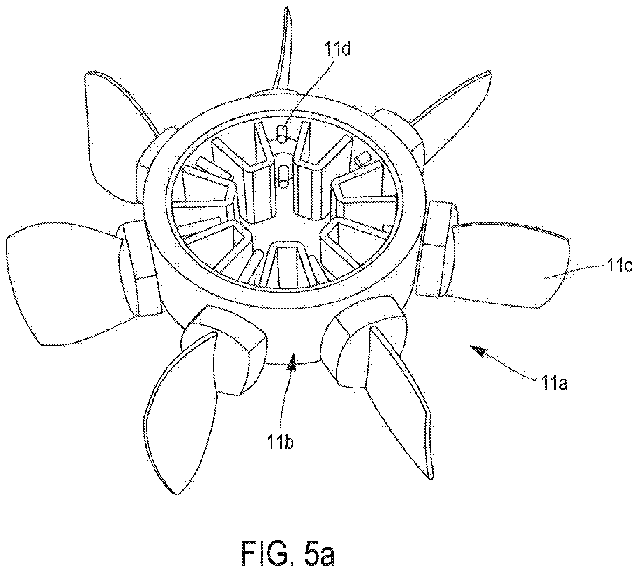

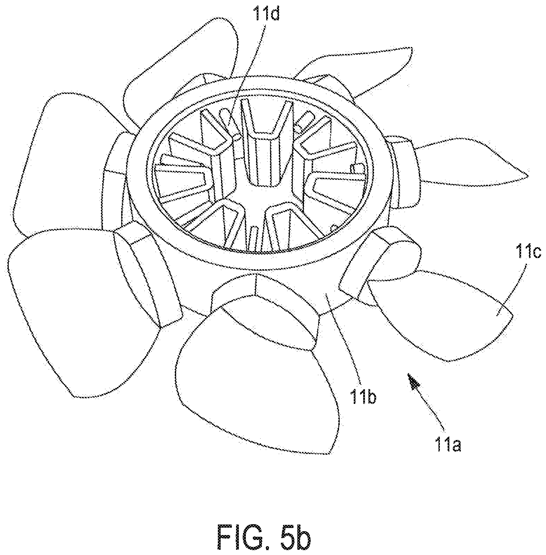

Selon une autre variante, on peut prévoir de disposer d'une turbine 11a dont les pales 11c peuvent être sélectivement orientées selon un angle de calage choisi, de manière à adapter à l'avance le dispositif à son environnement et au niveau de débit de fluide qui est susceptible de le traverser. A tire d'exemple, on a représenté sur les figures 5a et 5b une turbine 11a sur laquelle les pales 11c sont montées de manière mobile au moyeu 11b par l'intermédiaire d'un dispositif de fixation 11d réversible, et permettant d'orienter axialement les pales 11c avant de placer le rotor dans un dispositif conforme à l'invention.

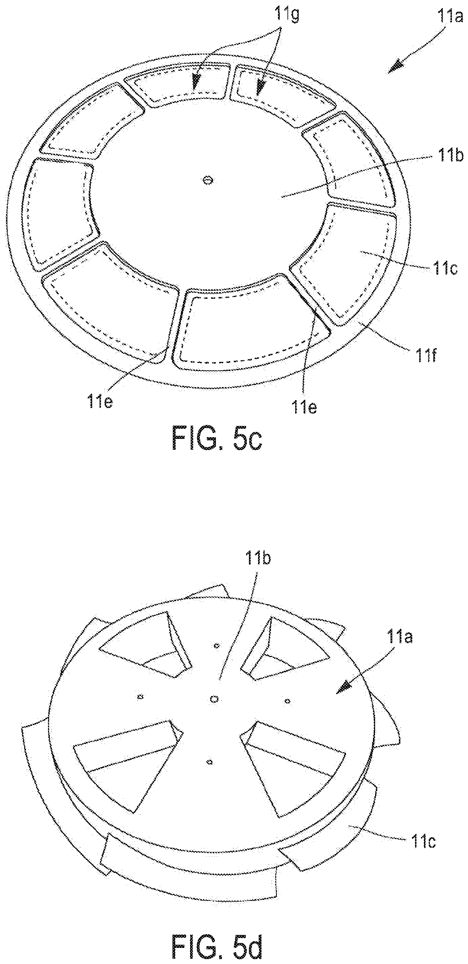

Selon une autre variante encore, les pales 11c peuvent être choisies en un matériau flexible ou souple, dont la forme et l'angle de calage est rendu variable selon le débit du fluide circulant dans le conduit. On a ainsi représenté sur la figure 5c, une telle turbine 11a, dont le moyeu 11b est relié par l'intermédiaire de bras 11e à une couronne de maintien 11f, de sorte à constituer des fenêtres 11g. Les pales 11c sont constituées d'un film plastique souple maintenu par le moyeu 11b, les bras 11e et/ou la couronne 11f. Des découpes (en pointillé sur la figure 5c) formées dans le film plastique, au niveau des fenêtres 11g, le long des bras 11e et/ou du moyeu 11b et/ou de la couronne 11f, définissent des languettes permettent au film de se déformer sous la pression du flux de fluide circulant dans le conduit, de manière à former des pales 11c dont l'angle de calage est rendu variable avec le débit de ce fluide.

On peut, en adoptant l'une des variantes qui viennent d'être décrites, contribuer à contrôler la turbine pour que, dans toutes les gammes de vitesse du fluide, par exemple entre 0,1m/s et 10 m/s, ou 0,1m/s à 4m/s, la turbine n'excède pas une vitesse de rotation de 1000 tour/min, ou de 800 tours/min voire de 400 tour/min.

Plus généralement, on peut prévoir de rendre la géométrie des pales variables, et d'ajuster cette géométrie aux conditions de l'écoulement du fluide, afin de favoriser en toute condition le développement d'un couple important et d'une vitesse de rotation faible.

Selon une première approche, la dimension de la turbine 11a est choisie pour occuper toute la section du conduit 14 quand celui-ci est de forme circulaire. Dans le cas d'un conduit d'une forme différente, le diamètre de la turbine 11a peut être choisi pour correspondre au diamètre du cercle inscrit dans la section du conduit 14. En occupant de la sorte une partie maximale de la section de passage du conduit 14, on tire profit au maximum de l'énergie disponible et fournie par le fluide.

Dans une variante, la dimension de la turbine 11a est choisie pour occuper une portion uniquement du conduit 14, la portion résiduelle pouvant être occultée de manière à constituer une restriction de la section du conduit et forcer une vitesse d'air plus importante à travers la turbine 11a.

Selon une autre variante, dont on donnera un exemple de mise en œuvre dans la suite de cet exposé, la portion résiduelle est laissée libre de manière à permettre la circulation d'une partie du fluide dans le conduit sans qu'il contribue à la mise en rotation de la turbine 11a. On limite ainsi la vitesse de rotation de la turbine, notamment lorsque la vitesse du fluide est susceptible d'être importante.

Selon l'invention, la portion résiduelle peut être sélectivement ouverte ou fermée par un rideau ou un volet.

L'invention n'est bien entendu pas limitée à la turbine 11a qui vient d'être décrite, bien que celle-ci ait montré un fonctionnement satisfaisant pour une vitesse d'écoulement aussi faible que 0,1 m/s, et qu'elle présente l'avantage d'un niveau d'émission sonore très faible, inférieur à 35dB(A) (pour une vitesse de fluide de 1m/s).

L'homme du métier dispose de nombreux outils et ouvrages pour concevoir une turbine 11a qui adoptera un régime de fonctionnement (c'est-à-dire un couple et une vitesse de rotation selon la vitesse et la nature du fluide) le plus adapté aux conditions particulières de fonctionnement du dispositif autonome 10 (nature du fluide, gamme de vitesse de fluide, dimension de la section de passage du conduit, impact des émissions sonores). On peut ainsi prévoir de fournir un dispositif muni d'une première turbine particulièrement adaptée pour être placée dans un conduit de ventilation (ou à l'extrémité de ce conduit) débouchant dans une cuisine et présentant donc un débit (et donc une vitesse de l'air) relativement important, et de fournir une deuxième turbine, différente de la première, particulièrement adaptée pour être placé dans un conduit de ventilation débouchant dans des toilettes et présentant donc un débit et une vitesse d'air relativement faible. Selon la destination du dispositif, on pourra choisir de l'équiper de la première ou de la seconde turbine. Dans tous les cas, la turbine favorise une rotation lente du rotor de manière à limiter les nuisances sonores et l'usure du dispositif.

En référence aux figures 3a et 3b, le rotor 11 comprend également une source permanente de champ magnétique 3 solidaire de la turbine 11a. En d'autres termes, la source de champ magnétique 3 est entraînée en rotation par la turbine 11a lorsque celle-ci est en mouvement. La source permanente de champ magnétique 3 forme la source de champ du générateur 1, conformément à ce qui a été présenté en introduction à cet exposé.

La source permanente de champ magnétique 3 définie un plan magnétique disposé perpendiculairement à l'axe de rotation de la turbine 11a. La source magnétique 3 ne génère pas nécessairement un champ confiné dans un plan, et il est tout à fait conforme à l'invention que le champ généré par la source occupe un volume s'étendant de part et d'autre d'un plan moyen. On pourra définir le plan magnétique dans ce cas comme ce plan moyen.

A titre d'exemple, la source 3 peut être formée d'une pluralité d'aimants 11e. Dans une configuration particulièrement avantageuse, les aimants 11e sont assemblés pour former un cylindre de Halbach, l'axe de ce cylindre étant aligné sur l'axe de rotation de la turbine. Comme cela est représenté sur les figures, les aimants 11e peuvent être maintenus entre eux par un corps 11d d'aspect général cylindrique, qui permet également d'assembler fixement la source magnétique 3 à la turbine 11a.

L'intérieur du cylindre de Halbach définit un logement dans lequel règne un champ de direction uniforme, et comprenant le plan magnétique.

La somme des moments magnétiques des aimants 11e formant le cylindre est nulle, l'interaction magnétique du cylindre avec un champ magnétique extérieur et donc nulle ou extrêmement réduite. Ainsi le champ magnétique terrestre ou une source magnétique disposée à proximité du dispositif ne créera pas ou peu de couple sur ce dernier. Cette propriété assure que le dispositif autonome 10 de l'invention puisse être disposé dans des conduits présentant des orientations quelconques (horizontale, ou verticale par exemple) et disposés près de sources de champ magnétiques sans modifier son fonctionnement ou sa performance.

Quelle que soit la forme prise par la source permanente de champ magnétique 3, la rotation du rotor conduit à former un champ magnétique tournant disposé au moins dans un plan perpendiculaire à l'axe de rotation de la turbine 11a.

### Le stator

Le dispositif autonome 10 selon l'invention comporte également un stator 12. Lorsque le dispositif autonome 10 est en fonctionnement dans un conduit 14, le stator 12 est fixement maintenu sur le conduit 14 par l'intermédiaire des moyens de fixation 13. Il n'est donc pas en mouvement.

Une fonction du stator 12 est de convertir l'énergie de mouvement rotatif du rotor 11 en des charges électriques suffisantes pour faire fonctionner le dispositif autonome 10. À cet effet, le stator 12 comprend un convertisseur magnéto-électrique 2 conforme à ce qui a été présenté dans le détail dans les paragraphes d'introduction de la description. Le convertisseur 2 présente donc un plan de référence et une variation de champ magnétique dans ce plan de référence conduit à une déformation mécanique susceptible de générer des charges électriques.

Le stator 12 comprend également un circuit de traitement 12a électriquement relié au convertisseur 2. Le circuit de traitement 12a est apte à exploiter les charges générées par le convertisseur 2 pour fournir une mesure d'une caractéristique de son environnement. Cet environnement est constitué par de l'air quand le dispositif autonome 10 est placé dans un conduit de ventilation.

Le stator 12 est assemblé au rotor 11 de sorte que le plan principal du convertisseur 2 réside dans le plan magnétique généré par la source 3. On forme de la sorte un générateur 1 apte à transformer le mouvement de rotation du rotor 11, dû à l'écoulement du fluide dans le conduit, en des charges électriques.

Lorsque le plan de référence du convertisseur 2 comprend une couche fortement magnétisable, comme c'est le cas des matériaux magnétostrictifs présentés dans la partie introductive de cette description, le convertisseur 2 est magnétiquement retenu dans le plan magnétique défini par la source 3. On constitue de la sorte un palier de butée magnétique qui supporte la poussée transmise par la turbine 11a.

Ce phénomène, qui conduit à maintenir le rotor 11 et le stator 12 dans des plans parallèles, peut être exploité pour permettre un assemblage facilement démontable du rotor 11 et du stator 12. On peut en effet facilement séparer le rotor 11 du reste du dispositif autonome 10 en appliquant un effort réduit et suffisant pour extraire le plan magnétique de sa position d'équilibre stable, aligné avec la couche fortement magnétisable.

Cette fonctionnalité de démontage simple du rotor 11 est particulièrement avantageuse. Elle permet par exemple de nettoyer le stator 11 et le rotor des graisses et des poussières qui pourraient s'y déposer. Elle permet également de facilement reconfigurer ou maintenir le dispositif, par exemple en remplaçant un premier rotor défectueux ou mal adapté, par un second rotor qui peut présenter des caractéristiques différentes du premier. On peut procéder à cette maintenance sans devoir extraire complètement le dispositif 10 du conduit 14.

Selon une autre caractéristique avantageuse de l'invention, le rotor 11 peut être muni d'un identifiant de rotor. Il peut s'agir d'une marque magnétique ou optique, d'un circuit RFID, ou de tout autre repère permettant d'identifier le rotor et ses caractéristiques. L'identifiant peut être repéré par un capteur optique, ou un capteur magnétique placé sur ou dans le stator 12, permettant d'identifier la présence du rotor 11 et de ses caractéristiques. Ces informations peuvent être utilisées par le dispositif autonome 10 pour élaborer la mesure.

Une caractéristique importante du dispositif autonome 10 selon l'invention est que le couple de freinage issu du couplage magnétique qui se forme entre le stator 12 et le rotor 11 au cours de la rotation du rotor 11, est d'intensité très réduite. Dans le cas d'un dispositif autonome 10 conçu pour mesurer au moins une caractéristique de l'air circulant dans un conduit de ventilation (et donc pour un générateur 1 pouvant occuper un volume de l'ordre de 1cm^3), ce couple de freinage est inférieur à 1 mN.m. Par ailleurs, ce couple est essentiellement constant et indépendant de la vitesse de rotation du rotor 11. Ce couple de freinage très faible contribue à permettre la rotation de la turbine même pour de très faible vitesse d'air, ou plus généralement de fluide.

La rotation du rotor 11 à faible vitesse peut également être favorisée en plaçant un arbre de rotation 12c entre un premier centreur 12b disposé sur le rotor 11 et un second centreur 12b' disposé sur le stator 12. L'arbre 12c a pour fonction de guider le mouvement de rotation et de bloquer le mouvement relatif du stator 12 et du rotor 11 dans le plan perpendiculaire à l'axe de rotation du rotor. On limite ainsi les frottements pouvant se produire entre ces deux éléments. L'arbre 12c et les centreurs 12b, 12b' sont alignés sur l'axe de rotation de la turbine 11a. La nature des matériaux formant les centreurs 12b, 12b' et l'arbre de rotation 12c est choisie pour sa résistance à l'usure par frottement. Il peut s'agir par exemple d'un axe en acier (inox ou amagnétique de préférence) ou en plastique (POM, Teflon...) associé à des centreurs en plastique (POM, Teflon...), en métal (bronze, laiton, ...) ou en pierre (rubis...). On peut également prévoir un système à roulement, tel qu'un système à roulement à billes pour faciliter la rotation du rotor.

Alternativement à cette configuration, on peut prévoir un dispositif permettant le centrage purement magnétique du stator 12 et du rotor 11 ; ou encore associer un dispositif de centrage magnétique à un palier de butée mécanique. Dans toutes ces configurations, on préserve le caractère facilement démontable de l'assemblage du stator (12) et du rotor (11).

Pour rendre le dispositif 10 très robuste, le stator 12 peut comprendre ou former un boîtier étanche dans lequel sont placés le convertisseur 2 et le circuit de traitement 12a. Le conduit, notamment lorsqu'il s'agit d'un conduit de ventilation, peut voir circuler de l'air très humide et on protège de la sorte les parties les plus fragiles du dispositif 10. Dans le mode de mise en œuvre alternatif dans lequel ce sont le convertisseur 2 et le circuit de traitement 12a qui sont placés dans le rotor, on pourra prévoir que cet élément soit constitué ou comprenne un boitier étanche. Avantageusement, l'assemblage du convertisseur au boitier étanche et au circuit de traitement est réalisé par l'intermédiaire de moyens permettant d'absorber au moins en partie les vibrations ou les émissions sonores qui peuvent être émises par le convertisseur, notamment lors de la déformation soudaine des matériaux qui se produit au moment du prélèvement des charges. On contribue ainsi à limiter les émissions sonores du dispositif au cours de son fonctionnement.

Pour que le stator 12 ne forme pas un élément gênant pour l'écoulement du fluide, on peut lui donner une forme suffisamment compacte pour être logé dans le moyeu 11b de la turbine 11a. C'est notamment le cas lorsque la turbine 11a dispose d'un moyeu 11b en forme de dôme, présentant une base large pour accueillir le stator 12, comme cela est représenté sur la vue en coupe de la figure X. On contribue ainsi à limiter ou éviter la perte de charge dans le conduit.

Pour des raisons économiques et de compacité, le convertisseur 2 et le circuit de traitement 12a peuvent être disposés sur un unique substrat 12d, par exemple de type PCB. Le substrat 12d peut être muni de pistes conductrices permettant de relier électriquement le convertisseur, par exemple au niveau de sa ou de ses bornes de connexion, et les autres éléments du circuit de traitement 12a.

Le circuit de traitement 12a, comme on l'a présenté dans la partie introductive de cette description, comprend un circuit de prélèvement des charges fournies par le convertisseur. Ce circuit peut être configuré pour, par exemple, prélever quatre pulsations énergétiques à chaque rotation du rotor 11. Le circuit de prélèvement peut comprendre un dispositif de stockage des charges électriques, tel qu'une batterie ou une capacité, afin de disposer d'une source d'énergie stable sur une durée de temps suffisant pour élaborer et fournir la mesure.

A titre d'exemple, la figure 4 représente un graphe de la puissance générée (axe des ordonnées de gauche) par un générateur formé d'un rotor et d'un stator conforme à ce qui vient d'être décrit. Ce dispositif a été conçu pour fournir une mesure relative à l'air circulant dans un conduit de 125mm de diamètre. On observe que pour des vitesses d'air compris entre 1m/s et 3,5 m/s (axe des abscisses), le stator est entraîné en rotation à une vitesse jusque 1200 tr/min environ. Cette vitesse permet de générer jusque 6 milliwatts de puissance. Ce niveau d'énergie est suffisant pour permettre le fonctionnement d'un dispositif autonome 10 conforme à l'invention, et notamment d'alimenter le circuit de traitement. Ce niveau d'énergie peut être également suffisant pour alimenter un éventuel actionneur, par exemple un restricteur ou augmenteur de flux dans le conduit, comme cela sera exposé plus en détail par la suite.

Avantageusement, le circuit de traitement 12a comprend un circuit de transmission des mesures élaborées. Il peut s'agir d'un circuit d'émission-réception radio, par exemple au format Bluetooth^{™}, Zigbee^{™}, Zwave^{™}, un protocole ENOCEAN^{™} ou encore Sigfox^{™}. La nature du format choisi est notamment dictée par la portée requise du circuit de transmission, et de la distance séparant le dispositif autonome d'un récepteur.

Le circuit de traitement 12a, a pour fonction générale de fournir au moins une mesure d'une caractéristique de son environnement. Quand il est disposé dans un conduit dans lequel circule un fluide, son environnement comprend le fluide circulant dans le conduit.

Ainsi, selon un premier aspect, le circuit de traitement 12a est configuré pour établir une mesure de la vitesse du fluide. Cette mesure peut être facilement établie à partir de la mesure de la vitesse de rotation du rotor 11 ou de la puissance générée, à l'aide d'une table ou d'une relation de corrélation liant la vitesse de rotation de la turbine ou la puissance générée à la vitesse du fluide. On trouve une telle relation représentée en pointillé sur le graphe représenté à la figure 4.

Le dispositif de traitement 12a est configuré pour déterminer la vitesse de rotation de la turbine/du stator. On pourra tirer profit des pulsations énergétiques générées par le convertisseur 2 à chaque fraction déterminée de tour de rotation du rotor 11 pour obtenir cette vitesse de rotation. Ainsi, la vitesse de rotation du rotor 11 peut être déterminée en décomptant le temps entre deux pulsations successives.

Avantageusement la mesure de la vitesse du fluide reposera sur la vitesse de rotation du rotor 11. Cette méthode de détermination est plus fiable que celle se basant sur la puissance fournie par le générateur 1. En effet, la puissance délivrée peut-être variable selon les conditions particulières de l'environnement (température, humidité, degré d'usure) qui affectent dans le temps l'efficacité du générateur, c'est à dire la relation liant la puissance générée et la vitesse du fluide. Ce n'est pas le cas d'une méthode reposant sur le décompte de temps entre deux pulsations énergétiques.

On notera également que la mesure de vitesse du fluide peut être fournie, lorsque le dispositif autonome 10 est placé dans le conduit, sans nécessairement équiper ce dispositif 10 d'un capteur spécifique. On tire en effet profit des propriétés du générateur 1 d'énergie pour parvenir à établir cette mesure.

La relation de corrélation pouvant être dépendante des caractéristiques de la turbine 11a ou plus généralement du rotor 11, le dispositif de traitement 12a peut être configuré pour sélectionner la table ou la relation de corrélation correspondant à la turbine 11a ou au rotor 11 effectivement placé sur le dispositif autonome 10. On pourra à cet effet faire usage de l'éventuel identifiant de rotor pour que cette sélection se fasse automatiquement, comme cela a été exposé dans un passage précédent de la description.

Pour réaliser les traitements qui viennent d'être évoqués, le circuit de traitement 12a comprend, sur un support 12d, des composants électroniques discrets ou intégrés permettant de mettre en œuvre les fonctions décrites. Avantageusement, le circuit de traitement 12a peut comprendre un ou une pluralité de calculateurs programmables tels qu'un microcontrôleur ou un microprocesseur dédié au traitement du signal.

Dans certains cas, il peut être avantageux de munir le dispositif autonome 10 d'au moins un capteur additionnel. On profite ainsi de l'énergie disponible pour fournir une pluralité de caractéristiques du fluide, ou d'autres informations non directement liées au fluide lui-même.

On pourra ainsi munir le dispositif autonome 10 d'un capteur de température, d'humidité, de CO2, de CO, ou de particules organiques volatiles afin de fournir des mesures caractéristiques du fluide. Quand le conduit est un conduit de ventilation par extraction, ces mesures peuvent correspondre à l'atmosphère régnant dans la pièce d'où l'air a été prélevé. On peut donc en déduire des informations exploitables pour la commande intégrée de la ventilation, du chauffage et/ou de la climatisation, voire même de l'éclairage d'une pièce, de l'ouverture/fermeture de volet ou tout autre commande domotique.

On peut également munir le dispositif autonome 10 de capteurs ne cherchant pas directement à mesurer une caractéristique du flux du conduit. Ainsi, et dans la mesure où le dispositif 10 est placé à proximité de l'extrémité du conduit, on peut prévoir de munir ce dispositif 10 d'un capteur de présence ou de température de la pièce dans laquelle le conduit débouche. C'est notamment possible lorsque le dispositif autonome est intégré à une bouche de conduit 15, fixée à l'extrémité d'un conduit, et disposant donc d'un accès direct à la pièce.

Ces capteurs peuvent être reliés au circuit de traitement 12a par des connexions filaires. Alternativement ou en complément, le dispositif autonome 10 de mesure peut également être relié à des capteurs par l'intermédiaire d'un circuit de réception sans fil du circuit de traitement 12a. Le dispositif 10 peut alors être relié à des capteurs placés directement dans la pièce dont on souhaite mesurer avec précision certaines caractéristiques.

Le dispositif autonome 10 peut être également relié à un dispositif de commande, tel qu'un téléphone mobile, une tablette ou un ordinateur ou toute autre forme de calculateur afin d'être configuré ou diagnostiqué. Cette liaison peut être assurée par le circuit de transmission du circuit de traitement 12a. Il peut s'agir par exemple de préciser les caractéristiques du rotor 11, dans le cas où le dispositif ne dispose pas de la fonctionnalité d'identification automatique qui a été décrite précédemment.

Pour gérer au mieux l'énergie disponible, il n'est pas nécessaire que toutes les caractéristiques de fluide soient établies et fournies à une fréquence fixe. On peut ainsi choisir d'établir une première caractéristique (par exemple la mesure de vitesse du fluide) selon une première fréquence de mesure, et d'établir une deuxième caractéristique (par exemple une mesure de concentration en CO2 ou de CO) à une seconde fréquence, inférieure à la première. D'une manière générale, la fréquence de la mesure d'une caractéristique est choisie pour être adaptée à la dynamique du phénomène mesuré.

Il n'est pas non plus nécessaire de procéder à la transmission d'une mesure directement après son établissement. Une mesure peut être temporairement enregistrée dans une mémoire du circuit de traitement 12a et transmise à une fréquence qui peut être différente de la fréquence de la mesure. La quantité, la nature et la fréquence des relevés et des transmissions peuvent également être déterminées automatiquement afin de tirer un profit maximum de l'énergie fournie par le générateur.

### Moyens de fixation

Les moyens de fixation 13 permettent de retenir le dispositif autonome 10 dans le conduit 14 dans une position de fonctionnement. Dans cette position, l'axe de la turbine 11a est parallèle à la direction d'écoulement du fluide. Préférentiellement, les moyens de fixation 13 doivent maintenir suffisamment le dispositif autonome 10 pour qu'il puisse être positionné dans le conduit quelle que soit l'orientation de ce conduit, par exemple horizontale ou verticale.

À titre d'exemple, les moyens de fixation 13 peuvent comprendre un cadre, relié au stator par des branches 13a, le cadre présentant un contour, une partie au moins la surface extérieure de ce contour pouvant prendre appui sur la surface interne du conduit 14. Par exemple, le cadre peut présenter un contour circulaire, dont le diamètre correspond au diamètre du conduit. Cette configuration est celle représentée sur les figures 3a et 3b.

Selon une variante particulièrement intéressante, le dispositif de fixation 13 comprend une bouche de conduit 15, et le rotor 11 et le stator 12 sont au moins en partie disposés dans la bouche de conduit 15. La bouche peut comporter un manchon 15a dont la forme et la dimension sont ajustées à la forme et à la dimension du conduit 14. En introduisant l'extrémité du manchon 15a dans le conduit 14, on fixe la bouche 15 sur le conduit 14. Le rotor 11 et le stator 12 peuvent être disposés, au moins en partie, dans le manchon 15a. On forme alors un dispositif autonome 10 qui peut être installé très simplement par un utilisateur en remplaçant une bouche de conduit existante par une bouche comprenant un dispositif autonome 10 conforme à l'invention.

Outre sa fonction de fourniture d'au moins une mesure, le dispositif autonome 10 selon l'invention peut également être configuré pour modifier ou contrôler une des caractéristiques du fluide ou de son environnement. À titre d'exemple, le circuit de traitement 12a peut être configuré, après analyse des mesures établies, pour automatiquement commander des moyens de chauffage, de climatisation ou de ventilation. Dans l'exemple particulier où le dispositif 10 est disposé dans un conduit de ventilation, il peut s'agir de commander l'ouverture d'une fenêtre si la mesure fournie indique une mauvaise qualité d'air, comme un excès d'humidité ou de CO2. Il pourrait également s'agir de commander un dispositif de chauffage d'une pièce si la mesure du flux d'air de ventilation révèle une température inférieure à une température de confort.

Le circuit de traitement 12a peut être également configuré pour commander d'autres types de dispositifs. Par exemple, il peut s'agir de la commande d'une alarme visuelle ou sonore dans le cas de détection d'une fumée.

Avantageusement, le dispositif commandé permettant de modifier ou contrôler une des caractéristiques du fluide ou de son environnement peut faire partie du dispositif autonome 10 lui-même. Il peut s'agir par exemple d'un dispositif mettant en œuvre des moyens visant à modifier l'écoulement du fluide circulant dans le conduit. On peut ainsi envisager de placer un volet ou un rideau dont le déplacement contrôlé permettrait de modifier la section de passage du conduit. Le volet ou le rideau peuvent être par exemple formés d'un matériau piézoélectrique (PVDF) dont on peut contrôler la déformation ou la forme par application d'une tension. Le volet et/ou le rideau peuvent être intégrés dans les moyens de fixation 13, le mouvement d'ouverture ou de fermeture du volet ou du rideau pouvant être guidé par le cadre d'appui.

Selon une première approche, le volet ou le rideau peut être disposé en série avec le rotor, c'est à dire en amont ou en aval de celui-ci. Dans ce cas, le dispositif autonome 10 peut alors réduire, voire même complètement interrompre, la circulation du fluide dans le conduit, et par exemple réduire ou interrompre la ventilation d'une pièce selon les circonstances établies par les mesures fournies.

Pour permettre ce mode de fonctionnement, on peut prévoir que le dispositif autonome comprenne un dispositif de commande intégré au circuit de traitement 12a. Avantageusement, on prévoira dans le dispositif de commande intégré (dans le cas où le circuit de traitement ne le prévoit pas déjà par ailleurs) un élément de stockage des charges électriques, tel qu'une batterie ou une capacité, pour pouvoir accumuler l'énergie nécessaire à la mise en œuvre du dispositif commandé. Le dispositif de stockage est notamment utile pour permettre la commande d'ouverture du dispositif et rétablir la circulation du fluide (car dans le cas où la circulation du fluide est interrompue, il n'y a plus de génération d'énergie possible). Alternativement, les dispositifs commandés peuvent être conçues pour être « normalement ouvert », c'est à dire qu'en l'absence d'une fourniture d'énergie, ces dispositifs prennent une configuration de repos dans laquelle la circulation du fluide n'est pas complètement interrompue. Quelle que soit la solution mise en œuvre, on dispose alors d'un dispositif autonome 10 et auto régulé.

Selon une autre approche particulièrement avantageuse, le volet ou le rideau peut être disposé en parallèle avec le rotor, c'est à dire qu'il peut occulter ou libérer un passage de fluide de dérivation, contournant les pales de la turbine. On peut de la sorte ouvrir un passage de circulation du fluide, sans que celui-ci ne favorise la mise en rotation du rotor. Ce mécanisme est particulièrement avantageux lorsque l'on souhaite préserver une vitesse de rotation faible du rotor, par exemple inférieure à 1000 tour/min ou 800 tour/min malgré un débit d'air relativement important. Comme on l'a déjà indiqué, une faible vitesse de rotation est favorable pour l'usure du dispositif et son émission sonore.

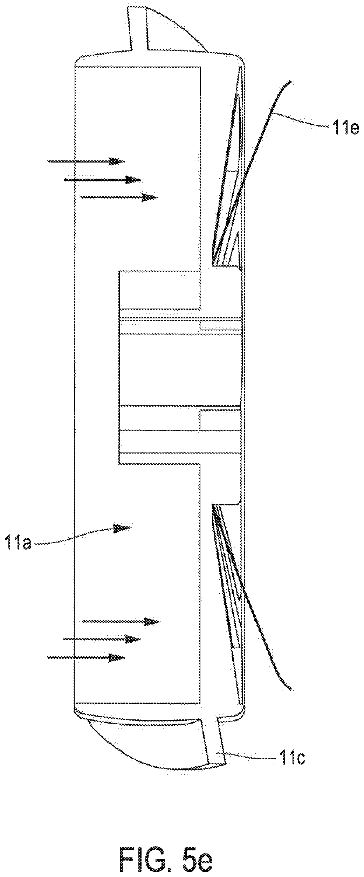

Il n'est pas nécessaire que l'ouverture du rideau ou du volet, dans cette autre approche soit pilotée. A ce titre, on présente en figure 5d (en perspective) et en figure 5e (en coupe transversale), un rotor 11a, dont le moyeu 11b est muni d'un rideau flexible 11e en plastique. Ce rideau 11e (particulièrement visible sur la figure 5e) est susceptible de se déformer sous l'effet du flux de fluide circulant dans le conduit de manière à dégager un passage permettant au fluide de circuler sans contribuer à l' entrainement en rotation du rotor. Plus le débit de fluide est important, plus le rideau tend à dégager une ouverture importante pour permettre au fluide de circuler dans ce passage de dérivation. On notera que ce mode de mise en œuvre permet de fournir un passage de dérivation sans affecter le débit du fluide dans le conduit. On pourra prévoir que le rideau soit précontraint, et qu'il ne se déforme que lorsque le flux excède une valeur déterminée.

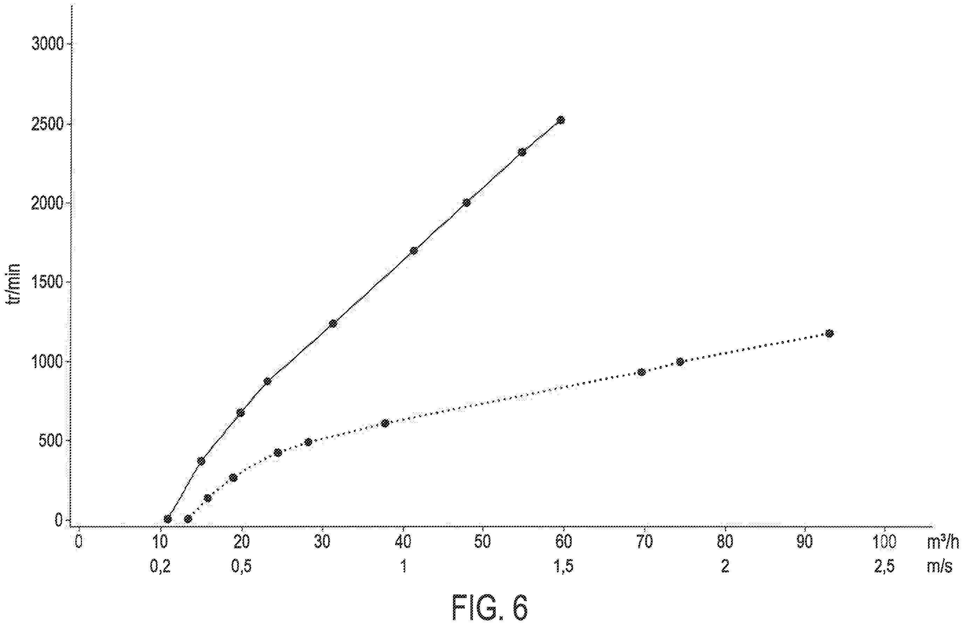

On a ainsi représenté sur la figure 6 la vitesse de rotation suivant le débit d'air d'une turbine placée dans un conduit de 125mm de diamètre présentant un moyeu central plein (trait plein) et d'une turbine présentant un moyen central présentant un tel rideau flexible (pointillé). On observe que la vitesse de rotation est notablement diminuée dans cette seconde configuration, pour tous les débits d'air. On note qu'il est possible de maintenir la vitesse de rotation sous 1000 tr/min pour une large plage de débit d'air. On a observé plus de 3dB(A) de réduction d'émission sonore de la seconde configuration comparativement à la première à 50m3/h.

### Système de commande.

Le dispositif autonome 10 de mesure d'une caractéristique d'un fluide circulant dans un conduit peut être employé dans un système de commande de la ventilation, de la climatisation et/ou du chauffage d'un bâtiment. Le bâtiment peut être un bâtiment d'habitation ou un bâtiment industriel ou de bureaux. Comme cela est usuel, ces bâtiments disposent d'un réseau de conduits dans lequel circulent des fluides, généralement de l'air, pour assurer les fonctions qui viennent d'être citées.

A titre d'exemple, le réseau peut être constitué d'un premier réseau de chauffage et de climatisation permettant d'injecter dans les pièces du bâtiment de l'air réchauffé ou refroidi, et d'un second réseau de ventilation permettant d'extraire de l'air de ces pièces afin de permettre son renouvellement. Selon un aspect avantageux, l'invention prévoit de placer au moins un dispositif autonome 10 dans l'un et/ou l'autre de ces réseaux.

Un système de commande conforme à l'invention comprend donc au moins un dispositif autonome 10 de mesure conforme à l'une des variantes qui viennent d'être exposées dans le détail. Le dispositif autonome 10 de mesure est disposé dans un conduit du réseau dans une configuration de fonctionnement à l'aide des moyens de fixation 13. De manière pragmatique, on disposera de préférence une pluralité de ces dispositifs autonomes 10, dans une pluralité de conduits, de manière à disposer de suffisamment d'informations pour piloter finement le niveau de confort et de qualité d'air du bâtiment. Il peut s'agir par exemple de placer un dispositif 10 dans chacun des conduits d'injection et d'extraction du réseau à proximité des pièces desservies dans le bâtiment.

Le système de commande conforme à l'invention comprend également au moins un dispositif de commande permettant d'ajuster l'état de ventilation, de climatisation ou de chauffage du bâtiment. Le dispositif de commande reçoit et traite les mesures fournies par le (ou les) dispositif autonome pour contrôler les moyens de chauffage, de climatisation ou de ventilation du bâtiment en vue d'atteindre un niveau de confort et de qualité d'air déterminé. Ce niveau peut être choisi par un résident, un occupant et/ou un gérant du bâtiment.

Selon une variante, le dispositif autonome 10 comporte des moyens visant à modifier l'écoulement du fluide circulant dans le conduit dans lequel il est placé. Le dispositif de commande est apte à contrôler ces moyens, par exemple pour augmenter ou réduire le débit d'air circulant dans un conduit de ventilation, de chauffage et/ou de ventilation. Le dispositif de commande peut alors, et dans une certaine mesure, contrôler l'état du bâtiment sans nécessairement faire appel ou solliciter les moyens disponibles de ventilation, de chauffage ou de climatisation. Le système de commande peut, dans cette variante, prévoir une position de repos total, consistant à activer les moyens visant à modifier l'écoulement du fluide pour interrompre totalement cet écoulement ; et éteindre ou mettre en veille les moyens de chauffages, de climatisation et/ou de ventilation du bâtiment, tels que les pompes, les échangeurs thermiques, ... On peut placer ainsi le bâtiment en veille pour des périodes prolongées. Alternativement on peut prévoir d'activer, par sécurité, l'extinction ou la mise en fonctionnement réduit des moyens de chauffages, de climatisation, et/ou de ventilation, lorsqu'un nombre important de dispositifs autonomes ont mise en œuvre les moyens visant à modifier, et notamment limiter, l'écoulement du fluide afin d'éviter une sollicitation excessive de ces moyens lorsque le débit d'air à faire circuler est limité.

Le système de commande peut également être couplé à d'autres capteurs de mesure, comme des capteurs de température d'une pièce ou des capteurs de présence dans une pièce, de manière à élaborer une commande sans se fonder uniquement sur les mesures fournies par le (ou les) dispositif autonome 10.

Le système de commande, et plus particulièrement le dispositif de commande, est avantageusement configuré pour fournir des informations de diagnostic ou de maintenance du réseau de conduits. Les informations fournies par les dispositifs autonomes 10, telles que les informations de débit, permettent d'identifier les fuites ou les pertes de charge, liées par exemple à un encrassement excessif ou un endommagement d'un conduit.

Comme on l'a vu précédemment, une partie au moins des fonctionnalités du dispositif de commande peut être intégrée au dispositif autonome. Dans ce cas, le circuit de traitement 12a du dispositif autonome 10 comprend un circuit de transmission ayant des capacités d'émission-réception, lui permettant d'échanger des informations. Ces informations peuvent par exemple être échangées avec d'autres dispositifs autonomes 10 du système, notamment celles concernant l'état de ventilation, de climatisation et/ou de chauffage du bâtiment. Le circuit de traitement 12a de chacun de ces dispositifs autonomes peut être configuré pour exploiter les informations reçues. On forme alors un système de commande distribué, ne nécessitant pas nécessairement un dispositif de commande présentant une partie centralisée ou un concentrateur pour son fonctionnement.

D'une manière générale, le dispositif de commande peut prendre la forme d'un calculateur. Lorsqu'il est au moins partiellement intégré dans un dispositif autonome, le calculateur peut-être celui du circuit de traitement 12a.

Lorsque le dispositif de commande comprend une partie centralisée ou un concentrateur, le calculateur peut-être un ordinateur, un téléphone portable ou une tablette. Le dispositif de commande centralisé ou le concentrateur n'est pas nécessairement localisé dans le bâtiment équipé du (ou des) dispositif autonome 10. On peut ainsi prévoir que les mesures fournies par le dispositif autonome 10 soient adressées, directement ou indirectement, au dispositif de commande centralisé ou au concentrateur par un réseau longue distance, tel que le réseau Sigfox^{™} ou le réseau Internet. On peut alors prévoir de commander, à partir d'un poste centralisé, l'état de ventilation, de chauffage et/ou de climatisation d'une flotte de bâtiments. On peut de la sorte établir une stratégie d'économie d'énergie à grande échelle, mettant en œuvre par exemple une stratégie de délestage permettant de réduire le niveau de confort dans certains créneaux horaires (nuit, week-end, vacances) afin de gérer au mieux la consommation énergétique des bâtiments.

Bien entendu l'invention n'est pas limitée au mode de mise en œuvre décrit et on peut y apporter des variantes de réalisation sans sortir du cadre de l'invention tel que défini par les revendications.

Bien que l'on ait pris dans certains exemples la mesure d'une caractéristique de l'air circulant dans un conduit de ventilation, l'invention n'est certainement pas limitée à cette application. Ainsi, le dispositif conforme à l'invention peut être employé pour fournir une mesure relative à un fluide circulant dans n'importe quel conduit d'un réseau de conduits d'un bâtiment. Il peut s'agir par exemple d'un conduit de climatisation, ou de chauffage dans lequel de l'air refroidi ou réchauffé circule.

Le dispositif 10 conforme à l'invention n'est pas non plus limité à un fluide gazeux. Il peut s'agir d'un fluide liquide, comme de l'eau, circulant dans une conduite d'alimentation.

Et l'invention n'est nullement limitée à une application dans le domaine du bâtiment. Elle peut être employée d'une manière générale pour établir la mesure d'une caractéristique d'un fluide quelconque circulant dans un conduit quelconque.

## Revendications

1. Dispositif autonome (10) de mesure d'au moins une caractéristique d'un fluide circulant dans un conduit (14) selon une direction d'écoulement, le dispositif autonome (10) comportant :
- un rotor (11) comprenant une turbine (11a) susceptible d'être entraînée en rotation par le fluide, le rotor étant assemblé à un stator (12) et la turbine (11a) étant formée d'un moyeu (11b) et d'une pluralité de pales (11c) fixées sur le moyeu (11b) ;
- des moyens de fixation (13) permettant de placer fixement le stator (12) dans le conduit ou à une extrémité du conduit, dans une configuration selon laquelle l'axe de rotation de la turbine (11a) est parallèle à la direction d'écoulement ;
- une source permanente de champ magnétique (3) définissant un plan magnétique perpendiculaire à l'axe de rotation de la turbine (11a);
- un convertisseur magnéto-électrique (2) présentant un plan de référence et apte à transformer une variation dans le plan de référence d'un champ magnétique en une déformation mécanique susceptible de générer des charges électriques ;
- un circuit de traitement (12a), électriquement relié au convertisseur (2), et apte à exploiter les charges électriques pour fournir une mesure d'une caractéristique de son environnement ;
l'un du convertisseur (2) et de la source (3) étant compris dans le stator (12), et l'autre étant compris dans le rotor (11); le stator (12) étant assemblé au rotor (11) de sorte que le plan de référence du convertisseur (2) réside dans le plan magnétique généré par la source (3), le dispositif (10) comprenant en outre des volets ou un rideau pour modifier la section de passage du conduit, les volets ou le rideau étant configurés ou pilotés pour limiter la vitesse de rotation du rotor en libérant un passage de circulation du fluide contournant les pales (11c) de la turbine (11a).

2. Dispositif autonome (10) selon la revendication précédente dans lequel la source (3) est comprise dans le rotor (11), solidaire de la turbine (11a) ; et dans lequel le convertisseur (2) est compris dans le stator (12).

3. Dispositif autonome (10) selon la revendication 1 dans lequel le convertisseur (2) et le circuit de traitement (12a) sont compris dans le rotor (11), solidaire de la turbine (11a) ; et dans lequel la source (3) est comprise dans le stator (12).

4. Dispositif autonome (10) selon l'une des deux revendications précédentes dans lequel le convertisseur (2) et le circuit de traitement (12a) sont disposés dans un boîtier étanche.

5. Dispositif autonome (10) selon la revendication 1 dans lequel les pales (11c) sont orientables ou constituées d'un matériau flexible.

6. Dispositif autonome (10) selon l'une des revendications précédentes dans lequel le moyeu (11b) comprend un rideau susceptible de dégager un passage de circulation du fluide.

7. Dispositif autonome (10) selon l'une des revendications précédentes dans lequel la turbine (11a) est conçue pour présenter un paramètre de rapidité inférieur à trois.

8. Dispositif autonome (10) selon l'une des revendications précédentes dans lequel la turbine (11a) présente une solidité comprise entre 0,7 et 1.

9. Dispositif autonome (10) selon l'une des revendications précédentes dans lequel l'angle de calage des pales (11c) est compris entre 15° et 45.

10. Dispositif autonome (10) selon la revendication précédente dans lequel l'angle de calage des pales (11c) est variable.

11. Dispositif autonome (10) selon l'une des revendications précédentes dans lequel le convertisseur (2) comprend dans son plan de référence une couche de matériaux magnétisable, et magnétiquement retenue dans le plan magnétique généré par la source (2) de sorte à former un assemblage démontable entre le rotor (11) et le stator (12).

12. Dispositif autonome (10) selon l'une des revendications précédentes dans lequel les moyens de fixation (13) comprennent une bouche de conduit (15), le rotor (11) et le stator (12) étant placés à l'intérieur de la bouche de conduit (15) .

13. Dispositif autonome (10) selon l'une des revendications précédentes comprenant au moins un capteur choisi parmi un capteur de température, un capteur de concentration en CO2 ou de CO, ou en particules organiques volatiles, un capteur d'humidité, un détecteur d'identifiant de turbine.

## Patentansprüche

1. Autonome Vorrichtung (10) zum Messen mindestens eines Merkmals eines Fluids, das in einer Leitung (14) entlang einer Strömungsrichtung zirkuliert, wobei die autonome Vorrichtung (10) umfasst:
- einen Rotor (11), der eine Turbine (11a) enthält, die von dem Fluid in Rotation versetzt werden kann, wobei der Rotor an einem Stator (12) montiert ist und die Turbine (11a) aus einer Nabe (11b) und einer Vielzahl von Schaufeln (11c) gebildet ist, die an der Nabe (11b) befestigt sind;
- Befestigungsmittel (13), die es ermöglichen, den Stator (12) fest in der Leitung oder an einem Ende der Leitung in einer Konfiguration anzuordnen, in der die Rotationsachse der Turbine (11a) parallel zur Strömungsrichtung ist;
- eine Permanentmagnetfeldquelle (3), die eine magnetische Ebene definiert, die senkrecht zur Rotationsachse der Turbine (11a) verläuft;
- einen magnetoelektrischen Wandler (2), der eine Referenzebene aufweist und eine Änderung in der Referenzebene eines Magnetfelds in eine mechanische Verformung umwandeln kann, die elektrische Ladungen erzeugen kann;
- eine Verarbeitungsschaltung (12a), die elektrisch mit dem Wandler (2) verbunden ist und die elektrischen Ladungen nutzen kann, um eine Messung eines Merkmals ihrer Umgebung zu liefern;
wobei entweder der Wandler (2) oder die Quelle (3) im Stator (12) und das andere im Rotor (11) enthalten ist; wobei der Stator (12) an dem Rotor (11) so montiert ist, dass die Referenzebene des Wandlers (2) in der von der Quelle (3) erzeugten magnetischen Ebene liegt, wobei die Vorrichtung (10) außerdem Klappen oder einen Vorhang enthält, um den Durchgangsabschnitt der Leitung zu verändern, wobei die Klappen oder der Vorhang so konfiguriert sind oder gesteuert werden, dass sie die Rotationsgeschwindigkeit des Rotors begrenzen, indem sie einen Durchgang für die Zirkulation des Fluids unter Umgehung der Schaufeln (11c) der Turbine (11a) freigeben.

2. Autonome Vorrichtung (10) nach dem vorhergehenden Anspruch, bei der die Quelle (3) in dem Rotor (11) enthalten ist, der mit der Turbine (11a) fest verbunden ist; und bei der der Wandler (2) in dem Stator (12) enthalten ist.

3. Autonome Vorrichtung (10) nach Anspruch 1, wobei der Wandler (2) und die Verarbeitungsschaltung (12a) in dem Rotor (11) enthalten sind, der mit der Turbine (11a) fest verbunden ist; und wobei die Quelle (3) in dem Stator (12) enthalten ist.

4. Autonome Vorrichtung (10) nach einem der zwei vorhergehenden Ansprüche, wobei der Wandler (2) und die Verarbeitungsschaltung (12a) in einem dichten Gehäuse angeordnet sind.

5. Autonome Vorrichtung (10) nach Anspruch 1, bei der die Schaufeln (11c) verstellbar sind oder aus einem flexiblen Material bestehen.

6. Autonome Vorrichtung (10) nach einem der vorhergehenden Ansprüche, wobei die Nabe (11b) einen Vorhang enthält, der einen Durchgang für die Zirkulation des Fluids freimachen kann.

7. Autonome Vorrichtung (10) nach einem der vorhergehenden Ansprüche, wobei die Turbine (11a) so ausgelegt ist, dass sie einen Schnelligkeitsparameter von weniger als drei aufweist.

8. Autonome Vorrichtung (10) nach einem der vorhergehenden Ansprüche, wobei die Turbine (11a) eine Festigkeit von 0,7 bis 1 aufweist.

9. Autonome Vorrichtung (10) nach einem der vorhergehenden Ansprüche, bei der der Anstellwinkel der Schaufeln (11c) von 15° bis 45 beträgt.

10. Autonome Vorrichtung (10) nach dem vorhergehenden Anspruch, bei der der Anstellwinkel der Schaufeln (11c) variabel ist.

11. Autonome Vorrichtung (10) nach einem der vorhergehenden Ansprüche, wobei der Wandler (2) in seiner Referenzebene eine Schicht aus magnetisierbarem Material enthält und in der von der Quelle (2) erzeugten magnetischen Ebene magnetisch gehalten wird, so dass eine lösbare Verbindung zwischen dem Rotor (11) und dem Stator (12) entsteht.

12. Autonome Vorrichtung (10) nach einem der vorhergehenden Ansprüche, bei der die Befestigungsmittel (13) eine Leitungsmündung (15) enthalten, wobei der Rotor (11) und der Stator (12) innerhalb der Leitungsmündung (15) platziert sind.

13. Autonome Vorrichtung (10) nach einem der vorhergehenden Ansprüche, die mindestens einen Sensor enthält, der aus einem Temperatursensor, einem Sensor für die Konzentration von CO2 oder CO oder flüchtigen organischen Partikeln, einem Feuchtigkeitssensor und einem Turbinenidentifikationsdetektor ausgewählt ist.

## Claims

1. Autonomous device (10) for measuring at least one characteristic of a fluid circulating in a channel (14) in a flow direction, the autonomous device (10) including:
- a rotor (11) comprising a turbine (11a) capable of being driven in rotation by the fluid, the rotor being assembled to a stator (12) and the turbine (11a) being formed by a hub (11b) and a plurality of blades (11c)fixed to the hub (11b);
- fixing means (13) allowing the stator (12) to be fixedly placed in the channel or at one end of the channel, in a configuration in which the axis of rotation of the turbine (11a) is in parallel with the flow direction;
- a permanent magnetic field source (3) defining a magnetic plane perpendicular to the axis of rotation of the turbine (11a);
- a magneto-electric converter (2) having a reference plane and able to transform a variation in the reference plane of a magnetic field into a mechanical deformation capable of generating electric charges;
- a processing circuit (12a) electrically connected to the converter (2) and able to utilize the electric charges in order to provide a measurement of a characteristic of its environment;
one of the converter (2) and the source (3) being in the stator (12), and the other of the converter and the source being in the rotor (11); the stator (12) being assembled to the rotor (11) such that the reference plane of the converter (2) resides in the magnetic plane generated by the source (3), the device (10) further comprising flaps or a curtain in order to modify the passage portion of the channel, the flaps or the curtain being configured or controlled in order to limit the speed of rotation of the rotor by opening a fluid circulation passage bypassing the blades (11c) of the turbine (11a).

2. Autonomous device (10) according to the preceding claim, wherein the source (3) is in the rotor (11) and integral with the turbine (11a); and wherein the converter (2) is in the stator (12).

3. Autonomous device (10) according to claim 1, wherein the converter (2) and the processing circuit (12a) are in the rotor (11) and integral with the turbine (11a); and wherein the source (3) is in the stator (12).

4. Autonomous device (10) according to either of the two preceding claims, wherein the converter (2) and the processing circuit (12a) are arranged in a sealed housing.

5. Autonomous device (10) according to claim 1, wherein the blades (11c) are adjustable or made of a flexible material.

6. Autonomous device (10) according to any of the preceding claims, wherein the hub (11b) comprises a curtain capable of relieving a fluid circulation passage.

7. Autonomous device (10) according to any of the preceding claims, wherein the turbine (11a) is designed to have a speed parameter of less than three.

8. Autonomous device (10) according to any of the preceding claims, wherein the turbine (11a) has a solidity of between 0.7 and 1.

9. Autonomous device (10) according to any of the preceding claims, wherein the pitch angle of the blades (11c) is between 15° and 45°.

10. Autonomous device (10) according to the preceding claim, wherein the pitch angle of the blades (11c) is variable.

11. Autonomous device (10) according to any of the preceding claims, wherein the converter (2) comprises, in its reference plane, a layer of magnetizable materials magnetically retained in the magnetic plane generated by the source (2) so as to form a removable assembly between the rotor (11) and the stator (12).

12. Autonomous device (10) according to any of the preceding claims, wherein the fixing means (13) comprise a channel mouth (15), the rotor (11) and the stator (12) being placed inside the channel mouth (15).

13. Autonomous device (10) according to any of the preceding claims, comprising at least one sensor selected from among a temperature sensor, a sensor for measuring the concentration of CO2 or CO or volatile organic particles, a humidity sensor, or a turbine identifier detector.
